# EUROPEAN PATENT APPLICATION

(11) **EP 2 656 792 A1**
(43) Date of publication of application: **30.10.2013**
(21) Application number: 11851428.0
(22) Date of filing: 22.12.2011
(51) Int. Cl.: A61B 8/06, A61B 8/00

(54) **ULTRASOUND DIAGNOSTIC APPARATUS AND ULTRASOUND DIAGNOSTIC APPARATUS CONTROL METHOD**

(30) Priority: 24.12.2010 JP 2010287297
(71) Applicant: Panasonic Corporation, Osaka 571-8501 (JP)
(72) Inventor: KAWABATA, Akihiro, 7F Twin 21 OBP Panasonic Tower 2-1-61 Shiromi, Chuo-ku, Osaka 540-6207 (JP); ITOH, Yoshihiko, 7F Twin 21 OBP Panasonic Tower 2-1-61 Shiromi, Chuo-ku, Osaka 540-6207 (JP); SUZUKI, Takao, 7F Twin 21 OBP Panasonic Tower 2-1-61 Shiromi, Chuo-ku, Osaka 540-6207 (JP)
(74) Representative: Grünecker, Kinkeldey, Stockmair & Schwanhäusser
(86) International application number: PCT/JP2011/007208
(87) International publication number: WO 2012/086207

(57) **Abstract**

An ultrasonic diagnostic apparatus according to the present invention is an apparatus to which a probe with transducers is connectible. The apparatus includes a controller **100** which performs transmission processing for sending out an ultrasonic wave toward a subject by driving the probe **1** and reception processing for generating a received signal based on the ultrasonic wave that has been reflected from the subject and received at the probe and which performs, if a predetermined condition based on the received signal is satisfied, automatic freeze processing for stopping at least one of the transmission processing and the reception processing. The controller **100** restricts the automatic freeze processing for a predetermined period of time after having accepted a predetermined operating event.

## Description

### TECHNICAL FIELD

The present invention relates to an ultrasonic diagnostic apparatus and a method for controlling the ultrasonic diagnostic apparatus.

### BACKGROUND ART

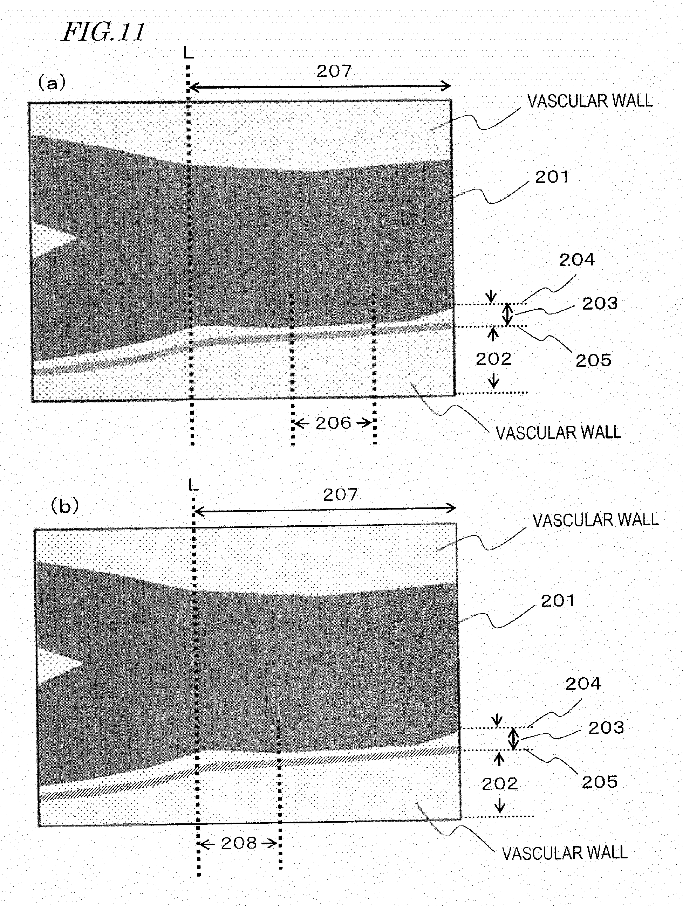

An ultrasonic diagnostic apparatus can capture a tomographic image representing an internal body tissue of a subject, and is currently used extensively to monitor the shape of an internal body tissue. Also, in diagnosing arterial sclerosis, an ultrasonic diagnostic apparatus is used to measure the intima-media thickness (which will be abbreviated herein as "IMT") of the carotid artery, which is an important index to detect initial signs of atherosclerosis. That is to say, IMT is the thickness of an intima-media complex of the vascular wall of the carotid artery, and the thickness of a layer seen between the vascular lumen **201** and the adventitia **202** (which will be referred to herein as "intima-media **203")** as shown in FIGS. **11(a)** and **11(b)****.** The ultrasonic diagnostic apparatus detects the boundary between the vascular lumen **201** and the intima (which will be referred to herein as a "lumen-intima boundary **204")** and the boundary between the media and the adventitia **202** (which will be referred to herein as a "media-adventitia boundary **205")** and calculates the distance between those two boundaries as the IMT.

In measuring the IMT, with an IMT measuring range **206** is set in the blood vessel running direction in which the carotid artery runs (which will be referred to herein as a "longer axis direction"), either the maximum thickness **max IMT** or the mean thickness **mean IMT** in that range is ordinarily measured as the IMT value as shown in FIG. **11(a)****.** For example, Non-Patent Document No. 1 recommends setting the IMT measuring range **206** 1 cm away from one far end (i.e., the end that is closer to the head) of the common carotid artery (CCA) **207** of the carotid artery. In the example illustrated in FIGS. **11(a)** and **11(b)****,** the portion on the right-hand side of the dotted line **L** is the common carotid artery, and the range shown in FIG. **11(b)** is the recommended IMT measuring range **208.**

Patent Document No. 1 discloses an ultrasonic diagnostic apparatus that measures the IMT automatically. The ultrasonic diagnostic apparatus of Patent Document No. 1 includes region setting means for setting a region to evaluate the reliability of the blood vessel's IMT measured, reliability calculating means for calculating the reliability of the IMT measured in the region that has been set by the region setting means and presenting the calculated reliability of the IMT measured on a display section, and IMT measuring means for measuring the IMT automatically in the region that has been set by the region setting means. Hereinafter, it will be described as an example how the ultrasonic diagnostic apparatus disclosed in Patent Document No. 2 may measure the IMT.

The IMT cannot be measured accurately unless the lumen-intima boundary **204** and media-adventitia boundary **205** of the blood vessel are clearly rendered by making the probe send and receive ultrasonic waves from/at around the center of cross section of the blood vessel that is defined to cross the longer axis direction of the blood vessel at right angles. Such a cross section will be referred to herein as a "shorter-axis cross section") and the longer axis direction means the direction in which the blood vessel runs. That is why first of all, the operator puts the probe on the subject's neck surface and adjusts the position and orientation of the probe surface, from/at which ultrasonic waves are sent or received, so that the ultrasonic wave can be sent and received from/at around the center of the shorter-axis cross section of the blood vessel of the carotid artery.

Once the position and orientation of the probe have been adjusted, the recommended IMT measuring range **208** shown in FIG. **11(b)** (i.e., the range set by the region setting means according to Patent Document No. 1) is set. Suppose in the initial state in which the probe is put at an appropriate position, the IMT measuring range has shifted from the recommended position to the right just like the IMT measuring range **206** shown in FIG. **11(a)****.** In that case, using a trackball, a touchscreen panel, or any other interface (i.e., region setting means) for setting the position of the IMT measuring range, the operator moves the IMT measuring range to the recommended IMT measuring range **208** shown in FIG. **11(b)****.** If a predetermined condition is satisfied after the IMT measuring range has been moved to the recommended one **208** with the probe held at an appropriate position, the IMT is measured automatically and an IMT value is calculated.

### CITATION LIST

### PATENT LITERATURE

Patent Document No. 1: Japanese Laid-Open Patent Publication No. 2010-022565

### NON-PATENT LITERATURE

Non-Patent Document No. 1: Journal of the American Society of Echocardiography February 2008, pp. 93 to 111

### SUMMARY OF INVENTION

### TECHNICAL PROBLEM

In the apparatus disclosed in Patent Document No. 1, the IMT calculated is presented on the display section. However, if the ultrasonic diagnostic apparatus needs to continue the measurement, the tomographic image presented on the display section keeps on being updated even after the IMT has been measured so that the latest tomographic image is always displayed. That is why if the tomographic image currently displayed is different from the one from which the IMT was obtained, the IMT is not associated with the tomographic image. In that case, it could be difficult for the operator to determine, by reference to the information displayed on the display section, whether or not the IMT has been measured properly. Also, if the IMT has once been measured successfully but currently cannot be measured properly because the probe has lost proper contact with the subject after that, then the latest measuring condition is not appropriate for making measurement, and therefore, neither an appropriate tomographic image nor a proper result of measurement can be presented. Nevertheless, as the IMT is still presented on the display section even in such a disturbed condition, the operator may sometimes find it unnatural. Furthermore, in a situation where the measurement needs to be continued, even if the IMT is presented, it could be difficult for him or her to decide when to finish the measurement.

Such a problem could arise not just when the IMT is measured but also when the length of a predetermined internal body tissue needs to be obtained in making various physical checkups or when a high definition tomographic image needs to be obtained as well.

An object of the present invention is to overcome at least one of these problems with the related art by providing a highly handy ultrasonic diagnostic apparatus and a method for controlling such an ultrasonic diagnostic apparatus.

### SOLUTION TO PROBLEM

An ultrasonic diagnostic apparatus according to the present invention is an apparatus to which a probe with transducers is connectible. The apparatus includes a controller which performs transmission processing for sending out an ultrasonic wave toward a subject by driving the probe and reception processing for generating a received signal based on the ultrasonic wave that has been reflected from the subject and received at the probe and which performs, if a predetermined condition based on the received signal is satisfied, automatic freeze processing for stopping at least one of the transmission processing and the reception processing. The controller restricts the automatic freeze processing for a predetermined period of time after having accepted a predetermined operating event.

An ultrasonic diagnostic apparatus controlling method according to the present invention is a method for controlling an ultrasonic diagnostic apparatus to which a probe with transducers is connectible. The method includes the steps of: (i) performing transmission processing for sending out an ultrasonic wave by driving the probe and reception processing for generating a received signal based on the ultrasonic wave that has been reflected from a subject and received at the probe; (iii) restricting, if a predetermined condition based on the received signal is satisfied, automatic freeze processing for stopping at least one of the transmission processing and the reception processing for a predetermined period of time after having accepted a predetermined operating event; and (iv) performing the automatic freeze processing after the predetermined period of time has passed.

Another ultrasonic diagnostic apparatus according to the present invention is an apparatus to which a probe with transducers is connectible. The apparatus includes an ultrasonic wave transmission and reception processing section which performs transmission processing for sequentially sending out ultrasonic waves one after another toward a subject by driving the probe and also performs reception processing for sequentially generating received signals one after another based on the ultrasonic waves that have been reflected from the subject and received at the probe; a tomographic image processing section which sequentially generates tomographic images based on the received signals and which determines whether or not the probe put on the subject is propagating an ultrasonic wave through the vicinity of the center of a short-axis cross section of a blood vessel included in the subject; a vascular wall thickness calculating section which calculates, based on the received signals, a value representing the vascular wall thickness of the blood vessel; a pulsation detecting section which determines whether the pulsating status of the blood vessel is detected properly or not and which outputs a signal at a predetermined timing during one cardiac cycle of the subject; a reliability decision section which outputs, depending on results of the decisions made by the tomographic image processing section and the pulsation detecting section, a signal indicating the degree of reliability of the vascular wall thickness value and the vascular wall thickness value; an image synthesizing section which synthesizes together the tomographic image and the signal representing the thickness of the vascular wall and which generates image data to be presented on a display section; and a control section which controls the ultrasonic wave transmission and reception processing section and the image synthesizing section so that in accordance with the signal indicating the degree of reliability, the ultrasonic wave transmission or reception processing is stopped and/or the tomographic image generated is put into a pause.

### ADVANTAGEOUS EFFECTS OF INVENTION

According to the present invention, if a predetermined condition about a received signal is satisfied, automatic freeze processing which stops at least one of transmission processing and reception processing is carried out. That is why if the length of a predetermined tissue is measured automatically or if a high-definition ultrasonic image is obtained when an IMT is measured or when any of various internal body tissues is inspected, the measurement is finished. Consequently, when any intended measurement is made automatically, the operator can find this apparatus very easy to handle.

In addition, by restricting the automatic freeze processing for a predetermined period of time after having accepted a predetermined operating event, the apparatus never enters the frozen state at an inappropriate timing even if measurement needs to be made once again by adjusting the measurement range after the intended measurement has gotten done once. Consequently, the handiness can be increased.

### BRIEF DESCRIPTION OF DRAWINGS

[FIG. **1****]** A block diagram illustrating an embodiment of an ultrasonic diagnostic apparatus according to the present invention.
[FIG. **2****]** A detailed block diagram of the ultrasonic diagnostic apparatus shown in FIG. **1****.**
[FIG. **3****] (a)** and **(b)** illustrate the relative position of the probe put on the subject with respect to a short-axis cross section of the blood vessel.
[FIG. **4****] (a)** is a schematic cross-sectional view illustrating a long-axis cross section of the carotid artery and **(b)** illustrates a waveform showing how the inside diameter of the carotid artery changes with time as blood pumps out of the heart.
[FIG. **5****]** A flowchart showing how the ultrasonic diagnostic apparatus shown in FIG. **1** operates.
[FIG. **6****]** Shows in detail a variation in the inside diameter of the carotid artery.
[FIG. **7****] (a)** shows a correlation between a carotid artery model waveform and an inside diameter variation waveform, while **(b)** shows how to extend the carotid artery model waveform on the time axis.
[FIG. **8****]** Shows the blood vessel inside diameter variation waveform, the IMT value variation waveform, and the ECG waveform.
[FIG. **9****]** Illustrates a carotid artery inside diameter variation waveform representing both a situation where the blood vessel is being inspected properly and a situation where the blood vessel is not being inspected properly.
[FIG. **10****] (a)** and **(b)** show how the reference value to decide whether or not to make an automatic freeze may be changed.
[FIG. **11****] (a)** and **(b)** illustrate the structure of the carotid artery and an IMT measuring range.

### DESCRIPTION OF EMBODIMENTS

Hereinafter, embodiments of an ultrasonic diagnostic apparatus and method for controlling such an ultrasonic diagnostic apparatus according to the present invention will be described in detail with reference to the accompanying drawings.

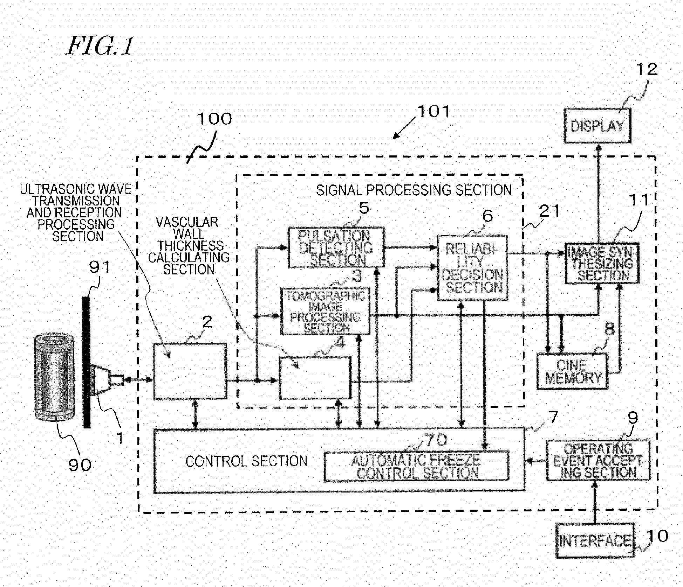

In the embodiment to be described below, the ultrasonic diagnostic apparatus measures the IMT of a subject. FIG. 1 is a block diagram illustrating such an ultrasonic diagnostic apparatus.

The ultrasonic diagnostic apparatus **101** includes a controller **100,** which includes an ultrasonic wave transmission and reception processing section **2,** a signal processing section **21,** a control section **7,** a cine memory **8,** an operating event accepting section **9,** and an image synthesizing section **11.** In this embodiment, the ultrasonic diagnostic apparatus **101** does not include a probe **1** but any probe **1** generally used may be connected to the apparatus **101.** However, the ultrasonic diagnostic apparatus 101 may include a probe **1.**

The ultrasonic wave transmission and reception processing section **2,** the operating event accepting section **9,** the cine memory **8,** and the image synthesizing section **11** may be implemented as a combination of known hardware components including various electronic parts. On the other hand, the signal processing section **21** may be implemented either by a software program or as a combination of hardware components. If the signal processing section **21** is implemented by a software program, it is recommended that the received signal generated by the signal processing section **21** be a digital signal. The control section **7** may be a combination of an arithmetic unit such as a microcomputer and a software program, for example.

The ultrasonic wave transmission and reception processing section **2** is designed so that the probe **1** is readily attachable and removable to/from the processing section **2.** The probe **1** has ultrasonic transducers, which sends out an ultrasonic wave toward a subject and which receives the ultrasonic wave that has been reflected from the subject and transforms it into an electrical signal.

By applying a drive pulse to the ultrasonic transducers of the probe **1** at a predetermined timing, the ultrasonic wave transmission and reception processing section **2** performs transmission processing to drive the probe **1** so that the probe **1** sends an ultrasonic wave. Also, by getting an electrical signal that has been obtained by transforming the ultrasonic wave received from the probe **1,** the ultrasonic wave transmission and reception processing section **2** performs reception processing, including amplification and detection of that electrical signal which need to be done to generate an ultrasonic tomographic image, thereby generating a received signal.

Normally, to obtain a tomographic image representing an internal body tissue of the subject from a region of interest including a region where the IMT is going to be measured, the ultrasonic diagnostic apparatus **101** sends out a number of ultrasonic waves and scans that region in which a tomographic image needs to be obtained with those ultrasonic waves. As a result of this scan, a received signal representing a tomographic image for one frame is obtained. And by performing this scan over and over again, several to several tens of frames of tomographic images can be generated per second. In this manner, the ultrasonic wave transmission and reception processing section **2** performs repeatedly and continuously transmission processing for one frame, thereby sequentially generating corresponding received signals one after another. Thus, the processing to be described below is carried out sequentially on the received signals generated one after another.

The signal processing section **21** receives the received signal that has been generated by the ultrasonic wave transmission and reception processing section **2** and performs various kinds of processing including generating an ultrasonic image, detecting the object of measurement and calculating a measured value. If the IMT needs to be measured, the signal processing section **21** suitably includes a tomographic image processing section **3,** a vascular wall thickness calculating section **4,** a pulsation detecting section **5** and a reliability decision section **6.**

The tomographic image processing section **3** receives the received signal that has been generated by the ultrasonic wave transmission and reception processing section **2** and subjects the received signal to coordinate transformation and other kinds of processing, thereby generating an ultrasonic image. In this embodiment, a tomographic image representing an internal body tissue of the subject is sequentially formed as a two-dimensional ultrasonic image. The tomographic image may be obtained by generating a luminance signal representing the amplitude (i.e., the signal intensity) of the received signal. Also, based on the tomographic image thus obtained, the tomographic image processing section **3** determines whether or not the position and orientation of the surface of the probe **1** that is put on the subject are appropriate ones and whether or not the ultrasonic wave being sent out from the probe **1** is going through the vicinity of the center of the short-axis cross section of the blood vessel that is the object of measurement.

As will be described in detail later, the vascular wall thickness calculating section **4** calculates the thickness of the vascular wall of the blood vessel as the IMT of the subject that is the object of measurement.

The pulsation detecting section **5** determines whether or not the pulsating status of the blood vessel is being detected properly, and outputs a signal at a predetermined timing during one cardiac cycle. That signal will be used at a reference time when the IMT is measured.

When measuring the wall thickness of the blood vessel that is the object of measurement in this example, the reliability decision section **6** determines, based on the respective outputs of (i.e., respective results of the decisions made by) the tomographic image processing section **3** and the pulsation detecting section **5,** whether the monitoring state is appropriate or not or whether the measured value is reliable enough as a result of measurement or not. That is to say, the reliability decision section **6** determines, based on the received signal, whether monitoring performed by the tomographic image processing section **3** and the pulsation detecting section **5** is appropriate or not or whether the measured value is reliable enough as a result of measurement or not. Also, the reliability decision section **6** receives the IMT value from the vascular wall thickness calculating section **4.** The result of the decision is output, along with the IMT measured value, as a signal indicating the degree of reliability of the IMT value.

The cine memory **8** receives the IMT value and a signal indicating the degree of reliability of that value from the reliability decision section **6** and stores them along with the signal representing the tomographic image that has been generated by the tomographic image processing section **3.** Although neither shown in FIG. **1** nor described in detail, this apparatus may also be configured so that the received signal generated by the ultrasonic wave transmission and reception processing section **2** is stored in the cine memory **8.**

The interface **10** is a device that is provided for the operator to operate this ultrasonic diagnostic apparatus **101** and may be a control panel, a trackball, a keyboard, a mouse, or a touchscreen panel, for example. The operating event accepting section **9** accepts the operating event that has been entered by the operator with the interface 10 and notifies the control section **7** of that event.

The control section **7** receives the operating event from the operating event accepting section **9** and controls respective blocks. Also, the control section **7** includes an automatic freeze control section **70,** which performs, based on a result of the decision made by the reliability decision section **6,** a control operation to enter the frozen state automatically, i.e., without having the operator make any operation to enter the frozen state by him- or herself, thereby determining the result of measurement to be the IMT value. Furthermore, only for a predetermined period of time after the operating event accepting section **9** has accepted a predetermined operating event, the automatic freeze control section **70** controls and restricts the automatic freeze. In the field of ultrasonic diagnostic apparatuses, the "frozen state" generally refers to a state in which ultrasonic wave transmission and reception processing is stopped and the image to display is put into a pause. In this description, however, the "frozen state" refers to a state in which at least one of stop of the ultrasonic wave transmission processing, stop of the ultrasonic wave reception processing, and putting the image to display into a pause is done. Also, "to restrict the automatic freeze" means not only delaying the apparatus' entering the frozen state automatically but also prohibiting the apparatus from entering the frozen state automatically as well.

In this manner, by determining the result of measurement as an IMT value and by doing at least one of stop of the ultrasonic wave transmission processing, stop of the ultrasonic wave reception processing, and putting the image to display into a pause, the tomographic image displayed becomes the same as the tomographic image from which the IMT was obtained. As a result, the operator never finds that tomographic image unnatural and can easily sense the timing to end the measuring process. Consequently, this apparatus provides great handiness without making the operator feel unnaturalness.

The image synthesizing section **11** is designed so that the display **12** is connectible to itself, and synthesizes together the result of the decision made by the reliability decision section **6** and the tomographic image that has been generated by the tomographic image building section **31** so that their synthetic image can be presented on the display **12.** The display **12** is a monitor which is connected to the image synthesizing section **11** and which displays its image sensor thereon.

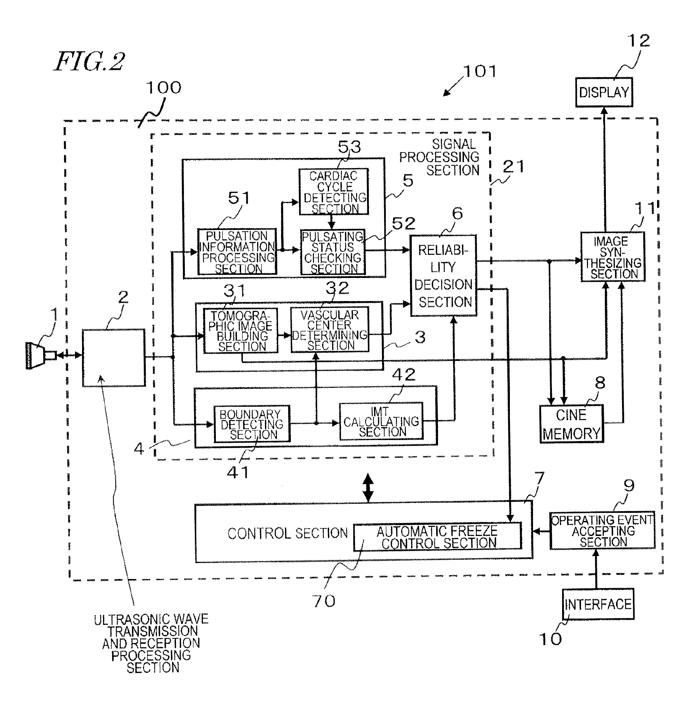

FIG. **2** is a block diagram illustrating a more detailed configuration for the ultrasonic diagnostic apparatus **101** shown in FIG. **1****.** Hereinafter, the configuration of the ultrasonic diagnostic apparatus **101** will be described in further detail with reference to FIG. **2****.** The components that have already been described with reference to FIG. **1** will not be described all over again.

As shown in FIG. **2****,** the vascular wall thickness calculating section **4** includes a boundary detecting section **41** and an IMT calculating section **42.** Based on the received signal obtained from the ultrasonic wave transmission and reception processing section **2,** the boundary detecting section **41** detects the two boundaries of the blood vessel under measurement (i.e., its lumen-intima boundary **204** and media-adventitia boundary **205)** in a range including the IMT measuring range (see FIGS. **11(a)** and **11(b)****).** The IMT calculating section **42** calculates, as the IMT, the distance between the lumen-intima and media-adventitia boundaries **204** and **205** that have been detected by the boundary detecting section **41.** In this case, max IMT is calculated as the IMT if the maximum distance in the IMT measuring range is adopted but mean IMT is calculated as the IMT if the mean distance in the IMT measuring range is adopted. However, these are only examples of the present invention. And either that max IMT or mean IMT is calculated as the final IMT value. However, this is just an example of the present invention. Alternatively, using multiple IMT values that have been measured within the IMT measuring range, various kinds of statistical processing may be carried out and the values thus obtained may be output.

The tomographic image processing section **3** includes a tomographic image building section **31** and a vascular center determining section **32.** As described above, the tomographic image building section **31** forms a tomographic image based on the received signal. Based on the tomographic image that has been formed by the tomographic image building section **31** and the boundaries of the blood vessel that have been detected by the boundary detecting section **41,** the vascular center determining section **32** determines whether or not the lumen-intima boundary **204** and media-adventitia boundary **205** are rendered clearly at the vascular boundary positions on the tomographic image and estimates the lengths of portions of the tomographic image in which the lumen-intima boundary **204** and media-adventitia boundary **205** are rendered clearly. This decision and this estimation are made based on the tomographic image. That is to say, this means that the vascular center determining section 32 makes that decision and that estimation based on at least one of the features (i.e., the signal intensity (or luminance) and the signal intensity (or luminance) distribution) of the received signal, of which a signal representing the tomographic image is formed. In addition, this also means that a topological feature such as the positions of the vascular boundaries needs to be estimated. In this manner, the vascular center determining section **32** determines whether or not the position and orientation of the surface of the probe **1** being put on the subject are appropriate and whether or not the ultrasonic wave being sent from the probe **1** is going through the vicinity of the center of a short-axis cross section of the blood vessel that is the object of measurement.

The pulsation detecting section **5** includes a pulsation information processing section **51,** a pulsating status checking section **52** and a cardiac cycle detecting section **53.** The pulsation information processing section **51** processes the received signals that have been generated by the ultrasonic wave transmission and reception processing section **2,** thereby extracting information from them in order to determine whether or not the blood vessel under measurement is pulsating. The pulsating status checking section **52** determines, based on the information that has been processed and extracted by the pulsation information processing section **51,** whether or not the blood vessel is pulsating. By reference to the information that has been processed and extracted by the pulsation information processing section **51,** the cardiac cycle detecting section **53** detects a particular timing during one cardiac cycle (e.g., the end of the diastolic phase that is a time when the heart that has contracted dilates to make the amount of the blood flow smallest).

In order to measure the IMT value as accurately as possible and with as good reproducibility as possible, the decisions by the vascular center determining section **32** and the pulsation detecting section **5** including the pulsation information processing section **51,** the pulsating status checking section **52** and the cardiac cycle detecting section **53** are made by seeing if the following two conditions are satisfied:

One of the two conditions is that the lumen-intima boundary **204** and the media-adventitia boundary **205** of the blood vessel are both rendered clearly. This decision is made by the vascular center determining section **32.**

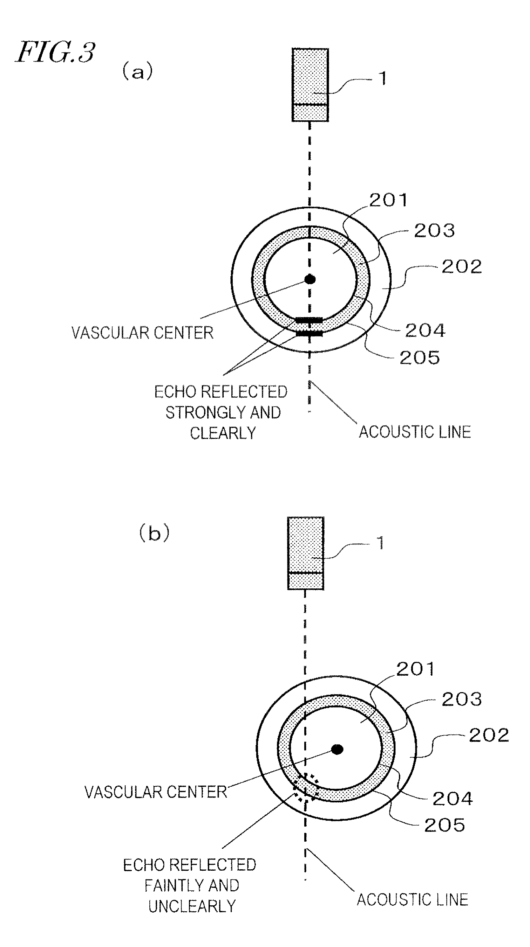

The IMT value is obtained by detecting the lumen-intima boundary **204** and the media-adventitia boundary **205** of the blood vessel and by measuring their thicknesses. For that reason, unless both of these two boundaries are rendered clearly, the IMT value cannot be measured accurately and with good reproducibility. And to render these two boundaries clearly, the position and orientation of the surface of the probe **1** being put on the subject need to be appropriate ones and the ultrasonic wave being sent out from the probe **1** needs to go through the vicinity of the center of a short-axis cross section of the blood vessel. That is to say, since the short-axis cross section of the blood vessel is almost circular, the probe **1** needs to be arranged so that the ultrasonic wave goes through the center of that circular cross section. This point will be described with reference to FIG. **3****.**

FIG**. 3** illustrates the relative position of the probe **1** put on the subject with respect to a short-axis cross section of the blood vessel. Specifically, FIG. **3(a)** illustrates a situation where the position and orientation of the surface of the probe **1** being put on the subject are appropriate ones. In that case, the ultrasonic echo that has been sent out from the probe **1** and reflected from an internal body tissue (i.e. an acoustic line) travels through the vicinity of the center of the short-axis cross section of the blood vessel. In this description, such a state will be sometimes referred to herein as "the probe is located right over around the center of the short-axis cross section of the blood vessel". Generally speaking, an ultrasonic echo is reflected from a boundary between two regions that have mutually different acoustic impedances (e.g., from a boundary between two tissues). In this case, the closer to 90 degrees the angle of incidence defined by the echo with respect to the boundary, the more strongly the echo will be reflected and the clearer the reflected echo signal will be. That is why in the state illustrated in FIG. **3(a)****,** strong and clear reflected echoes are obtained from both of the lumen-intima and media-adventitia boundaries **204** and **205.**

On the other hand, FIG. **3(b)** illustrates a situation where the surface of the probe **1** put on the subject faces an appropriate orientation but is not located at an appropriate position. In that case, the acoustic line of the ultrasonic echo transmitted from, and received at, the probe **1** does not pass through the vicinity of the center of the short-axis cross section of the blood vessel. In the state shown in FIG. **3(b)****,** the ultrasonic echo will not be incident perpendicularly onto the two boundaries of the blood vessel and only faint and unclear reflected echoes are obtained after all. As a result, the lumen-intima and media-adventitia boundaries **204** and **205** may be rendered as blurred and indistinct ones or the lumen-intima boundary may not be rendered at all.

For that reason, to render both of the lumen-intima and media-adventitia boundaries **204** and **205** of the blood vessel clearly, the position and orientation of the surface of the probe **1** need to be adjusted appropriately so that the probe **1** is located right over around the center of the short-axis cross section of the blood vessel.

As described above, the vascular center determining section **32** determines whether or not the first condition is satisfied. Specifically, by seeing if the lumen-intima and media-adventitia boundaries **204** and **205** are rendered clearly where those vascular boundaries should be located on the tomographic image, the vascular center determining section **32** can determine whether the probe **1** put on the subject is located right over around the center of the short-axis cross section of the blood vessel.

More specifically, by seeing if the tomographic image data representing the locations of the vascular boundaries detected and their surrounding sites has a portion in which the luminance rises from one side of the detected lumen-intima boundary **204** closer to the vascular lumen **201** toward the intima-media complex **203,** or a portion in which the luminance rises from one side of the detected media-adventitia boundary **205** closer to the intima-media **complex 203** toward the adventitia **202,** or a portion in which the luminance falls between the lumen-intima and media-adventitia boundaries **204** and **205** detected as shown in FIGS. **11(a)** and **11(b)****,** the decision section **32** can determine whether or not the lumen-intima and media-adventitia boundaries **204** and **205** are rendered clearly at the vascular boundary locations on the tomographic image. In this manner, a decision can be made about whether the first condition is satisfied or not.

In this case, the vicinity of the center of a short-axis cross section of the blood vessel where the two boundaries can be rendered clearly may be defined by the distance between the acoustic line shown in FIG. **3(a)** and the center of the cross section of the blood vessel, which may be 0.5 mm or less. But the distance will vary according to the subject, the precision of measurement of the ultrasonic diagnostic apparatus and the condition of measurement, and should not always be exactly equal to that value.

As described above, the length of a portion of the tomographic image in which the lumen-intima and media-adventitia boundaries **204** and **205** are rendered clearly may also be used as a criterion to determine whether the probe **1** put on the subject is located right over around the center of the short-axis cross section of the blood vessel. In that case, to make the decision, the two boundaries described above should be rendered clearly in ideally all, or at least a certain percentage, of the IMT measuring range (see FIGS. **11(a)** and **11(b)****).** For example, supposing the IMT measuring range has a length of 1 cm and the certain percentage is 75%, if those two boundaries are rendered clearly in at least 7.5 mm out of the overall length of 1 cm, then it can be determined that the blood vessel is caught properly around its center and that the first condition is satisfied.

The other condition is that the IMT which varies according to the pulsation be measured at a predetermined timing. At that time, the pulsation is detected by the cardiac cycle detecting section **53** and the pulsating status checking section **52** determines, by reference to the information that has been processed and extracted by the pulsation information processing section **51,** whether or not accurate information can be obtained about the blood vessel.

The blood vessel will contract and the vascular wall thickness will change to varying degrees according to the volume or rate of the blood flow running inside itself. That is why the IMT value changes according to the timing of measurement. For that reason, unless the IMT is measured at a predetermined timing, the IMT value cannot be measured accurately and with good reproducibility.

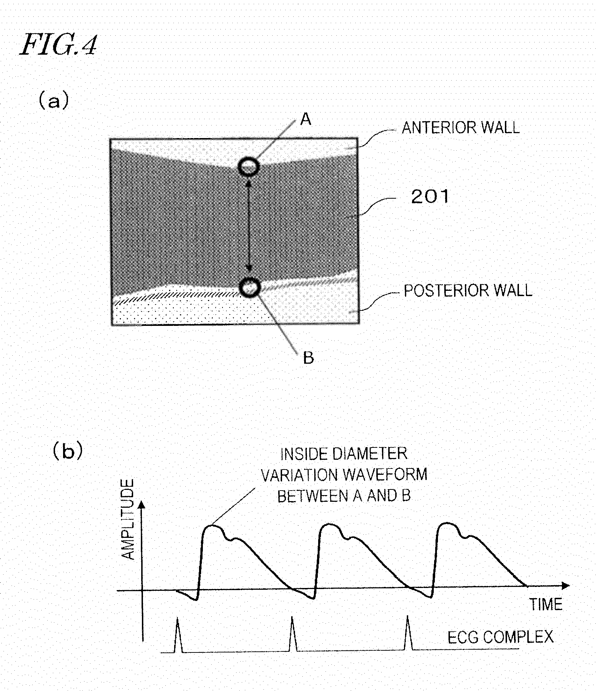

This point will be described in detail with reference to FIG. **4****.** Specifically, FIG. **4(a)** illustrates a cross section of the carotid artery in the longitudinal direction (which will be referred to herein as a "long-axis cross section") and FIG. **4(b)** illustrates a waveform showing how the inside diameter of the carotid artery changes with time as blood pumps out of the heart.

As the blood flow rate increases and the blood pressure rises in the systolic phase of the heart, the inside diameter of the blood vessel in that systolic phase (i.e., the distance between the two points **A** and **B** in the anterior and posterior walls shown in FIG. **4(a)****)** increases and the vascular wall thickness decreases. On the other hand, as the blood flow rate decreases and the blood pressure falls in the diastolic phase of the heart, the inside diameter of the blood vessel in that diastolic phase decreases and the vascular wall thickness increases. That is why as the vascular wall thickness varies synchronously with the heartbeat, the IMT value also changes according to when to measure it. Also, since the inner wall of the blood vessel varies with time as blood is pumped out of the heart, the pulsed waveform such as the one shown in FIG. **4(b)** is observed.

FIG. **4(b)** also shows the R-wave trigger timings of the ECG, which is usually used to detect the end of the diastolic phase of the heart. As shown in FIG. **4(b)****,** the inside diameter of the blood vessel once decreases immediately after an R-wave trigger timing of the ECG but increases steeply after that and then gradually returns to the original value. That is why in one cardiac cycle, the thickness of the vascular wall becomes maximum when the inside diameter of the blood vessel once decreases a predetermined amount of time after the end of the diastolic phase of the heart.

It is thought to be ideal to measure the IMT when the thickness of the vascular wall becomes maximum. That is why if the IMT is measured when the thickness of the vascular wall becomes maximum, the IMT can be measured accurately and with good reproducibility because the IMT is measured at a predetermined timing during one cardiac cycle.

In this embodiment, to detect the best timing to measure the IMT easily, the pulsating status checking section **52** determines, by reference to the information that has been generated by the pulsation information processing section 51 based on the received signal, whether or not there is pulsation. Also, by reference to the information that has been processed and extracted by the pulsation information processing section **51,** the cardiac cycle detecting section **53** detects and outputs a timing that is predetermined amount of time later than an R wave of the ECG described above.

The pulsating status checking section **52** outputs not only the signal indicating that timing, which has been received from the cardiac cycle detecting section **53,** but also a result of the decision about whether or not there is pulsation to the reliability decision section **6.**

In the embodiment described above, the cardiac cycle detecting section **53** is configured to detect the predetermined timing based on the received signal. However, the cardiac cycle detecting section **53** may also be configured to detect the predetermined timing using a normal ECG. In that case, the cardiac cycle detecting section **53** shown in FIG. **2** does not get the information that has been processed and extracted by the pulsation information processing section **51** (i.e., there is no need to provide any path leading from the pulsation information processing section **51** to the cardiac cycle detecting section **53)** and the ECG is connected to the cardiac cycle detecting section **53.**

As described above, the control section **7** includes the automatic freeze control section **70,** which performs a control to enter the frozen state automatically based on a result of the decision made by the reliability decision section **6** and determines a result of the measurement to be the IMT value. Since the IMT value can be measured by processing the received signal, no tomographic image needs to be presented on the display **12** at this point in time. In that case, however, as the operator cannot see that the frozen state has been entered, he or she can be notified of that either by indicating that with an icon put on the screen of the display **12** or by providing some notification means such as sounding an alarm. Optionally, such notification means may also be used even when an image is displayed.

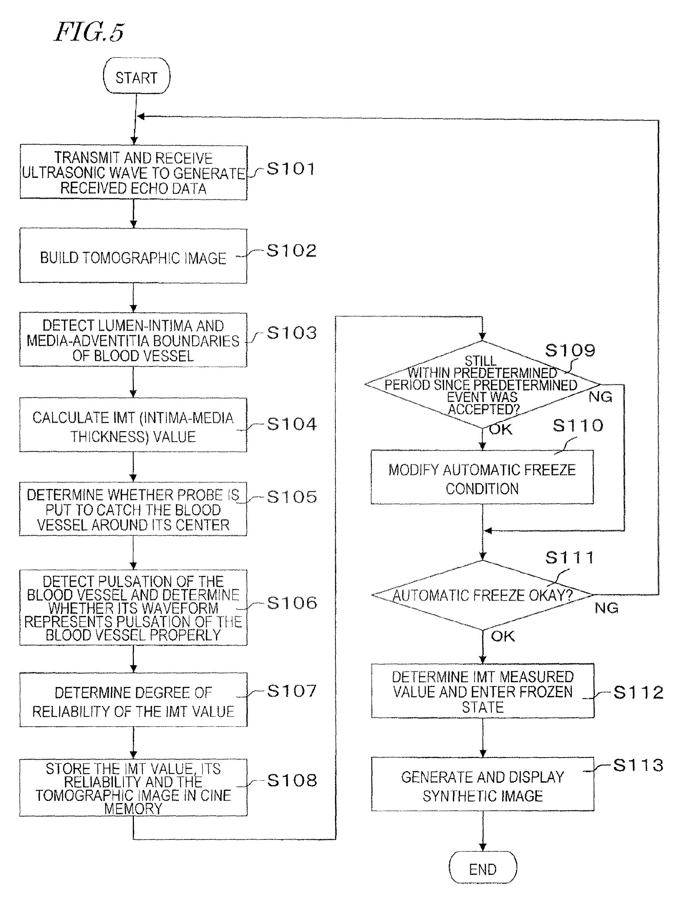

Hereinafter, it will be described with reference to FIG. **5** how this ultrasonic diagnostic apparatus **101** operates. FIG. **5** is a flowchart showing a typical operation of the ultrasonic diagnostic apparatus **101.**

First of all, in Step **S101,** the ultrasonic wave transmission and reception processing section **2** performs transmission and reception controls on ultrasonic signals, thereby transmitting an ultrasonic wave with the probe **1** driven and receiving, at the probe **2,** the ultrasonic wave that has been reflected form the subject. And just like a normal ultrasonic diagnostic apparatus, the ultrasonic wave transmission and reception processing section **2** performs ordinary signal processing on that reflected ultrasonic wave received to generate a received signal (received echo data).

Next, in Step **S102,** the tomographic image building section **31** processes the received signal, thereby building a tomographic image. The tomographic image data generated in this processing step may be a subject's visceral organ or any of various other objects. In this example, however, an image representing the subject's blood vessel (and an image representing his or her carotid artery, in particular) and its data are supposed to be processed mainly.

Meanwhile, the received signal is also output from the ultrasonic wave transmission and reception processing section **2** to the vascular wall thickness calculating section **4** and the pulsation detecting section **5.**

Subsequently, in Step **S103,** the boundary detecting section **41** of the vascular wall thickness calculating section **4** detects the lumen-intima and media-adventitia boundaries **204** and **205** of the blood vessel based on the amplitude and phase of the received signal that has been supplied from the ultrasonic wave transmission and reception processing section **2,** and outputs their location information to the vascular center determining section **32** and the IMT calculating section **42.** This processing step is carried out on each measuring point in the region of interest (ROI) of the image that has been defined in advance. In measuring the IMT, the ROI is normally defined so as to correspond with the IMT measuring range (see FIGS. **11(a)** and **11(b)****).**

Thereafter, in Step **S104,** based on the location information of the lumen-intima and media-adventitia boundaries **204** and **205** that have been detected by the boundary detecting section **41,** the IMT calculating section **42** calculates the thickness of the intima-media complex **203** (i.e., the IMT value).

In Step **S105,** the vascular center determining section **32** determines, based on the tomographic image provided by the tomographic image building section **31** and the boundary location information provided by the boundary detecting section **41,** whether or not the received signal representing the blood vessel being currently caught by the probe 1 has been obtained from around the center of the blood vessel.

Meanwhile, the received signal is also output from the ultrasonic wave transmission and reception processing section **2** to the pulsation detecting section **5.**

Next, in Step **S106,** the pulsation detecting section **5** checks the pulsating status of the blood vessel that is the object of measurement and determines whether or not its waveform represents the pulsation of the blood vessel correctly. Specifically, first, the pulsation information processing section **51** sets measuring points **A** and **B** on the anterior and posterior walls of the blood vessel under measurement as shown in FIG. **4(a)** and analyzes the amplitude and phase of the received signal, thereby tracking the motion of the measuring points **A** and **B.** Since the artery contracts and dilates repeatedly as the heart beats, the distance between these measuring points **A** and **B** also varies periodically as shown in FIG. **4(b)****.** That is why its periodic variation is detected as a waveform representing a variation in the inside diameter of the blood vessel. In this manner, simply by putting a probe on the subject without making any special connection between an electrocardiograph and the subject, such a waveform representing the variation in the inside diameter of the blood vessel can be obtained easily.

Next, by seeing if this inside diameter variation waveform obtained by the pulsation information processing section **51** has a pulse waveform, the pulsating status checking section **52** determines whether or not the inside diameter variation waveform represents the pulsation of the blood vessel properly.

This decision can be made by paying attention to a simple feature quantity of the inside diameter variation waveform (which will be referred to herein as "Method (1)") or examining how much the inside diameter variation waveform agrees with a reference (or model) waveform (which will be referred to herein as "Method (2)").

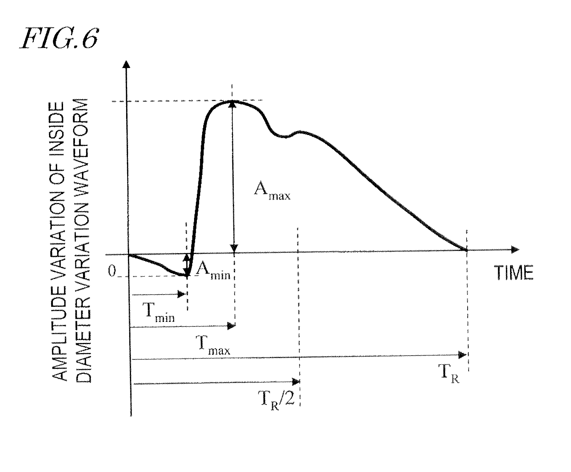

Method (1) will be described with reference to FIG. **6****,** which shows in detail a variation in the inside diameter of the carotid artery. In that case, the pulsating status checking section **52** examines, by reference to the waveform representing the variation in the inside diameter of the carotid artery, whether or not either the amplitude or a peak time falls within a normal human being's range. Examples of parameters of the feature quantity for use to make the decision may include:
· maximum and minimum amplitudes, which are respectively identified by Aₘₐₓ and Aₘᵢₙ in FIG. **6****;**
· time **Tₘₐₓ** when the amplitude becomes the maximum one **Aₘₐₓ** ;
· time **Tₘᵢₙ** when the amplitude becomes the minimum one **Aₘᵢₙ**
· one cardiac cycle **T_{R}**

As for these parameters, the present inventors confirmed via experiments that if the waveform shown in FIG. **6** had an Aₘₐₓ of slightly less than 1 mm, a negative Aₘᵢₙ value, and a T_{R} of about 1 second and if Tₘᵢₙ<Tₘₐₓ was satisfied, for example, the pulsating status could be checked out. That is why if all of these conditions are satisfied, then it can be determined that the waveform represents the pulsation properly.

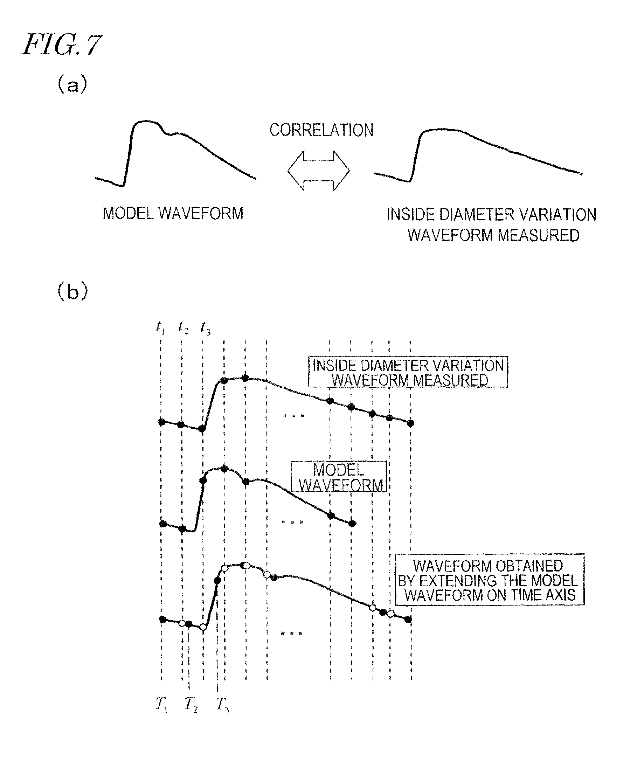

Next, Method (2) will be described with reference to FIG. 7. Specifically, FIG. **7(a)** shows a correlation between a carotid artery model waveform and an inside diameter variation waveform, while FIG. **7(b)** shows how to extend the carotid artery model waveform on the time axis.

First off, a reference model waveform is defined and the degree of matching between that waveform and the inside diameter variation waveform thus obtained is determined by calculating their correlation coefficient. In this case, the model waveform may be defined by collecting the data of the inside diameter waveforms of multiple persons in advance.

More specifically, a coefficient representing the degree of correlation between the model waveform and the inside diameter variation waveform thus obtained (which will be referred to herein as a "measured waveform") is calculated. If the model waveform and the measured waveform have mutually different time lengths, then the correlation coefficient may be calculated by extending or shortening the model and measured waveforms along the time axis so that those two waveforms have the same time length. FIG. **7(b)** illustrates a situation where the measured waveform has a longer time length than the model waveform. As one period (i.e., one cardiac cycle) of a model waveform disagrees in most cases with that of a subject's inside diameter variation waveform, the model waveform is extended or shortened along the time along with respect to the inside diameter variation waveform measured as shown in FIG. **7(b)****.** Next, a coefficient representing the degree of correlation between the model waveform that has been extended or shortened and the inside diameter variation waveform measured is calculated. And if the correlation coefficient is equal to or greater than a predetermined threshold value, then it can be determined that the waveform represents the pulsation properly.

In the example shown in FIG. 7**(b),** if one cardiac cycle of the inside diameter variation waveform of a subject does not agree with that of the model waveform, the model waveform is supposed to be extended or shortened along the time axis with respect to the inside diameter variation waveform measured. However, the same effect can be achieved even if the inside diameter variation waveform measured is extended or shortened along the time axis with respect to the model waveform.

The pulsating status may be checked out by using either only one of these two methods or both of them in combination. If only one of the two is adopted, the processing time can be shortened. On the other hand, if both methods are adopted, then the degree of matching between the two waveforms can be estimated more accurately.

This decision method is a waveform-based one. To obtain a more accurate IMT value, however, the timing of measurement is no less important as described above. That is why the timing representing the end of a diastolic phase should be detected.

The cardiac cycle detecting section **53** receives the blood vessel inside diameter variation waveform from the pulsation information processing section **51,** detects a time when the amplitude rises steeply (i.e., a timing when the inside diameter increases significantly) in the inside diameter variation waveform, and defines a time that is a predetermined amount of time before that timing to be the end of a diastolic phase.

As described above, it is a point in time around the end of one diastolic phase when the IMT value becomes maximum. That is why by detecting the point in time around the end of one diastolic phase, the IMT value can be measured at the best timing.

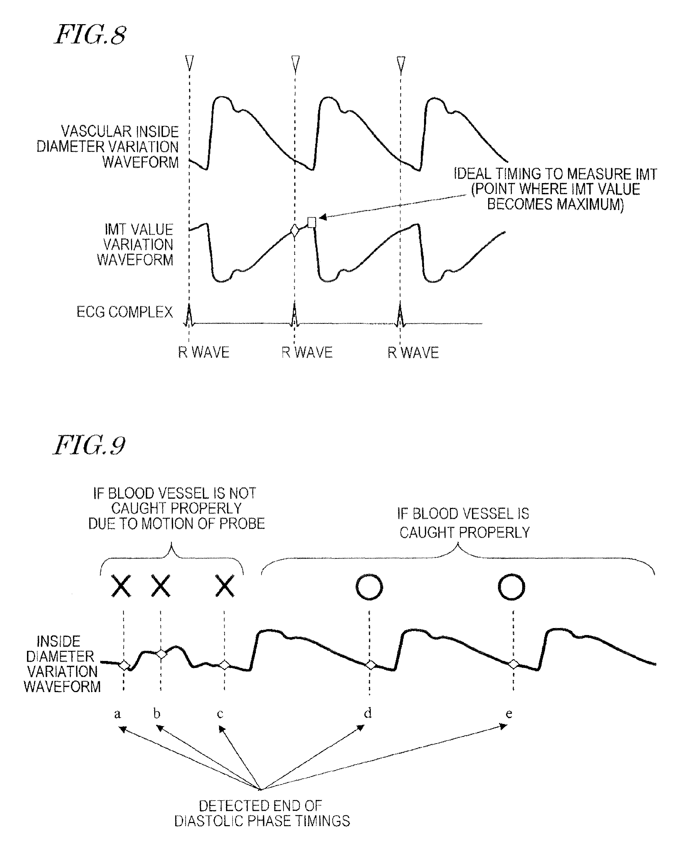

FIG. **8** shows the blood vessel inside diameter variation waveform, the IMT value variation waveform, and the ECG waveform with their time axes aligned with each other. Strictly speaking, the ideal timing when the IMT value becomes maximum is later by a predetermined amount of time than the end of the diastolic phase (corresponding to the R wave timing on an electrocardiogram) as shown in FIG. **8****.** That is why by determining the time to measure the IMT value with that time delay taken into account, the measurement can get done with even more likelihood. According to this method, the best time to measure the IMT can be detected easily even without using a device such as an ECG.

It should be noted that in one cardiac cycle, the best time to measure the IMT is not always the end of the diastolic phase but may also be set to be any other timing according to the processing time delay or processing method. Then, the apparatus of the present invention can be used even more universally.

FIG. **9** illustrates a waveform representing both a situation where the blood vessel is being inspected properly and a situation where the blood vessel is not being inspected properly.

In FIG. **9****,** the end of the diastolic phase is detected at respective points in time **a** through **e.** Among these points in time, the blood vessel is not caught properly at the former three points in time **a, b** and **c** but is caught properly at the latter two points in time **d** and **e.**

At the points in time **d** and **e,** the pulsating status has been checked out appropriately and the end of the diastolic phase has been detected properly by examining the inside diameter variation waveform. However, if a point in time that is not the end of the diastolic phase has been detected by mistake at the points in time **a, b** and c due to an inappropriate movement of the probe during the inspection for the purpose of finding the blood vessel, then it can be seen, by examining the inside diameter variation waveform, that the blood vessel is not caught properly.

That is to say, the pulsating status checking section **52** makes reference to both the result of examination on the inside diameter variation waveform that has been obtained by the pulsation information processing section **51** and the time indicating the end of the diastolic phase that has been detected by the cardiac cycle detecting section **53,** thereby deciding, with more accuracy, that the blood vessel is pulsating (i.e., the blood vessel is caught properly with the probe).

Next, in Step **S107,** the reliability decision section **6** examines the result obtained by the vascular center determining section **32** and the pulsating status that has been detected and checked by the pulsation detecting section **5,** thereby determining the degree of reliability of the IMT value that has been calculated by the IMT calculating section **42.** Specifically, the reliability decision section **6** determines the degree of reliability of the IMT value by seeing if the blood vessel that is currently caught as the object of inspection is in an appropriate state to determine the result of IMT measurement. The reliability decision is made based on a result of the decision that has been made by the vascular center determining section **32** and a result of the decision that has been made by the pulsating status checking section **52.**

More specifically, if the vascular center determining section **32** has decided that the probe **1** is put in a right position to catch the blood vessel around its center, the reliability decision section **6** regards the IMT value obtained as a highly reliable one. Likewise, if the pulsating status checking section **52** has decided that the pulsation of the blood vessel is being measured properly, the reliability determining section **6** also regards the IMT value obtained as a highly reliable one. And if these two conditions are satisfied, the reliability decision section **6** decides that the IMT value obtained is reliable enough to use as the final result of measurement.

Alternatively, instead of determining whether that value is a reliable one or not, the pulsating status checking section **52** and the vascular center determining section **32** may output a multi-level value representing the degree of probability. Specifically, the vascular center determining section **32** may calculate an estimated value indicating the degree of probability that the probe **1** is catching the blood vessel around its center. Meanwhile, the pulsating status checking section **52** may calculate an estimated value indicating the degree of probability that the pulsation of the blood vessel is being measured properly. Based on these estimated values that have been calculated by the vascular center determining section **32** and the pulsating status checking section **52,** the reliability decision section **6** may calculate an estimated value indicating the degree of reliability of the IMT value obtained.

In this case, the estimated value calculated by the vascular center determining section **32** may be set so that the greater the length of that portion of the tomographic image where the lumen-intima and media-adventitia boundaries **204** and **205** are rendered clearly, the larger the estimated value or that the more steeply the luminance of the tomographic image rises or falls around the lumen-intima and media-adventitia boundaries **204** and **205** detected, the larger the estimated value. Meanwhile, the estimated value calculated by the pulsating status checking section **52** may be set so that the greater the degree of correlation between the model waveform and inside diameter variation waveform measured, the larger the estimated value.

The reliability decision section **6** can make a decision based on either only the result obtained by the vascular center determining section **32** or just the one obtained by the pulsating status checking section **52.** If both of these two results are relied on, the accuracy of the decision can be increased. Nevertheless, depending on the situation of the inspection, the decision can also be made based on only of those two results. That is why the given software programs may be selectively used appropriately according to the application or cost of the apparatus, the quantity of signals to process and the signal processing ability of the controller **100.**

In Step **S108,** the cine memory **8** gets the IMT value and its degree of reliability from the reliability decision section **6** and stores them along with the tomographic image that has been formed by the tomographic image building section **31.**

Finally, the automatic freeze control section **70** determines, based on a result of the decision that has been made by the reliability decision section **6,** whether or not the condition to make an automatic freeze is satisfied. If the answer is YES, the automatic freeze control section **70** performs processing to enter the frozen state automatically (in Steps **S109** to **S112).**

To begin with, in Step **S109,** the automatic freeze control section **70** determines whether or not it is still within a predetermined period of time since the operating event accepting section **9** has accepted a predetermined operating event. If the answer is YES, the automatic freeze control section **70** modifies the condition for making an automatic freeze in Step **S110** in order to restrict the automatic freeze in the decision step **S111** later. In this case, the "predetermined period of time" may be 5 seconds after a predetermined operating event was accepted or after the operating event ended, for example. Optionally, the predetermined period may be set adaptively according to the type of the given operating event.

Examples of predetermined operating events to restrict the automatic freeze include: terminating (canceling) the frozen state; moving or changing the size of a range in which the ultrasonic wave transmission and reception processing section **2** performs ultrasonic wave transmission and reception processing; moving or changing the size of the region of interest (ROI) to be processed by the vascular wall thickness calculating section **4;** changing the depth range of the tomographic image generated by the tomographic image building section **31;** changing parameters (including a scan line density, whether parallel reception is performed or not, the number of lines used for the parallel reception, a transmission frequency, a transmission power, and a transmission interval) about the transmission and reception processing performed by the ultrasonic wave transmission and reception processing section **2;** changing parameters (including a gain, a dynamic range, whether filter processing needs to be performed or not, or a property of the filter processing) about the signal processing performed by the signal processing section **21;** and clearing the data stored in the cine memory **8**.

By restricting the automatic freeze after the operating event of terminating the frozen state has gotten done, in a situation where the frozen state was entered automatically once but has been terminated by the operator for further adjustments, it is possible to prevent the apparatus from entering the frozen state automatically again before the adjustments are done. Also, as described above, the recommended IMT measuring range (see FIGS. **11(a)** and **11(b)****)** is located 1 cm away from the far end (closer to the head) of the common carotid artery **207** of the carotid artery and the region of interest (ROI) to be processed by the vascular wall thickness calculating section **4** is set with respect to that IMT measuring range. However, by restricting the automatic freeze after moving or changing the size of the ROI, the automatic freeze can be avoided while the IMT measuring range is having its location and size adjusted into recommended ones.

Furthermore, by restricting the automatic freeze after the depth range of the tomographic image formed by the tomographic image building section **31** has been changed, the automatic freeze can be avoided while the depth range of the tomographic image is being adjusted.

Alternatively, after those parameters about the transmission and reception processing performed by the ultrasonic wave transmission and reception processing section **2,** including a scan line density, whether parallel reception is performed or not, the number of lines used for the parallel reception, a transmission frequency, a transmission power, and a transmission interval, have been changed, the automatic freeze may be restricted. In this description, the "scan line density" refers herein to the degree of narrowness of the distance between scan lines when ultrasonic wave transmission and reception scanning is carried out. The "parallel reception" refers herein to reception processing to be carried out on multiple scan lines in parallel in response to a single ultrasonic pulse transmitted. The "number of lines used for the parallel reception" refers herein to the number of scan lines that are used simultaneously to perform the reception processing. The "transmission frequency" refers herein to the frequency of the ultrasonic pulses to transmit. The "transmission power" refers herein to the amplitude of the ultrasonic pulse to transmit. And the "transmission interval" refers herein to the time intervals at which a series of ultrasonic pulses are transmitted. As a result, it is possible to avoid making an automatic freeze while these parameters about the transmission and reception processing are being changed.

Still alternatively, after those parameters about the signal processing performed by the signal processing section **21,** including a gain, a dynamic range, whether filter processing needs to be performed or not, or a property of the filter processing, have been changed, the automatic freeze may also be restricted. In this description, the "gain" refers herein to the magnitude of amplification of the received signal. The "dynamic range" refers herein to the range of the values of received signals to be represented as an image when the received signals are turned into a tomographic image or any other image. The "filter processing" refers herein to various kinds of filter processing, including a low-pass filter, a high-pass filter, a noise cutting spatial filter, and a filter between multiple frames of an ultrasonic image, which are applied to either the received signals or data obtained while the signals are being processed. As a result, it is possible to avoid making an automatic freeze while these parameters about the signal processing are being changed.

Still alternatively, by restricting the automatic freeze after the operating event involving clearing the data that is stored in the cine memory 8 has gotten done, the automatic freeze can be avoided while any operation involving clearing the data that is stored in the cine memory is being performed.

Some examples of predetermined operating events for restricting the automatic freeze have been described. Among those operating events, the automatic freeze may be restricted either after only one of them has gotten done or after multiple ones or even all of them have gotten done. Optionally, the automatic freeze may also be restricted after not any of these operating events but some other operating event has gotten done.

The processing step **S110** of modifying a condition for making the automatic freeze may get done either by disabling the automatic freeze altogether (which will be referred to herein as "Method (a)") or by tightening the condition for making the automatic freeze (which will be referred to herein as "Method (b)").

According to Method (a), irrespective of the result obtained by the reliability decision section **6,** the automatic freeze is prohibited absolutely in the next processing step **S111**. This method can be used no matter whether the reliability decision section **6** needs to decide whether the IMT value is a reliable one or not or needs to calculate an estimated value indicating the degree of reliability of the IMT value. Anyway, by adopting this method, it is possible to prevent the automatic freeze from turning ON and interfering with the operability while various kinds of adjustments are being made.

On the other hand, Method (b) is applicable to a situation where the reliability decision section **6** calculates an estimated value indicating the degree of reliability of the IMT value. And if the reference value for use to decide in the next processing step **S111** whether or not to make an automatic freeze is made stricter (i.e., if it is not until the estimated value indicating the degree of reliability of the IMT value exceeds a predetermined reference value that the freeze is enabled), then the reference value is changed into a higher one so that the estimated value does not exceed the reference value easily.

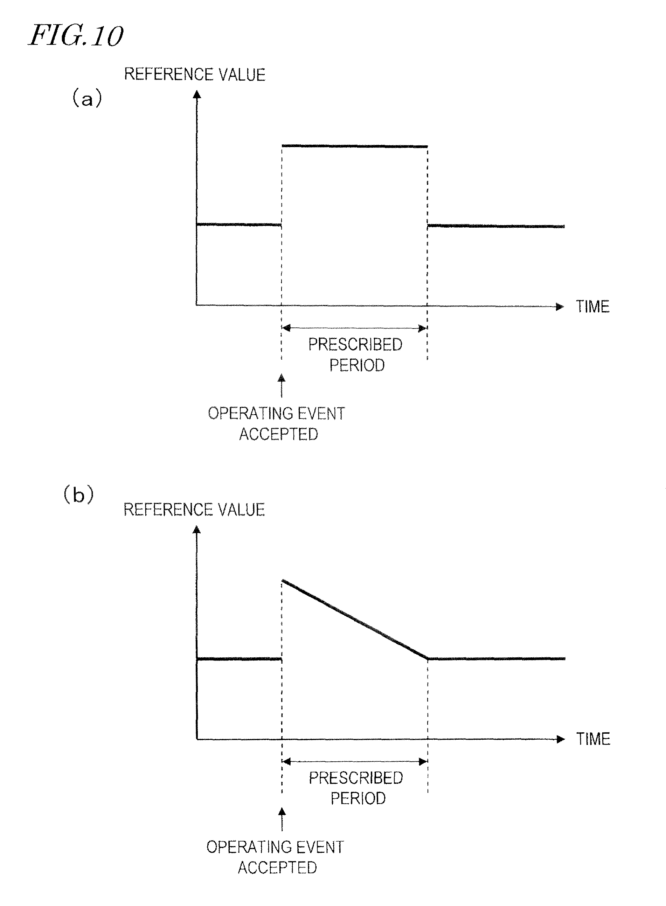

FIG. **10** shows how the reference value may be changed according to this Method (b). Specifically, FIG. **10(a)** shows how the reference value changes with time in a situation where the reference value is changed into a value that is greater than the original one by a predetermined quantity only for a prescribed period of time after the operating event has been accepted. In that case, the automatic freeze is not set off easily during an operation (e.g., while various adjustments are being made). As a result, it is possible to avoid a situation where the automatic freeze is set off unintentionally during an operation to affect the operability. In addition, if the IMT value has so high a degree of reliability as to exceed the reference value that has been changed into a larger value, the automatic freeze can be made by adopting that IMT value as a measured value even during an operation.

FIG. **10(b)** shows an exemplary situation where after the reference value has once been changed into a larger value than the original one when the operating event is accepted, the reference value is gradually decreased until the original condition before the change is restored. In that case, the shorter the amount of time it has passed since an operation such as various kinds of adjustments got done, the less likely the automatic freeze is set off. As a result, it is possible to avoid a situation where the automatic freeze is set off unintentionally during the operation to affect the operability. In addition, if the IMT value has a sufficiently high degree of reliability, the longer the amount of time it has passed since the operation was made, the more easily the automatic freeze can be set off by adopting that IMT value as a measured value even during an operation.

Next, in Step **S111,** the automatic freeze control section **70** determines, based on a result of the decision made by the reliability decision section **6,** whether or not to make an automatic freeze. If the reliability decision section **6** should determine whether the given IMT value is a reliable one or not, the automatic freeze is made if the answer is YES. On the other hand, if the reliability decision section **6** should calculate the degree of reliability of the IMT value as an estimated value, then the estimated value is compared to a predetermined reference value, and the automatic freeze is made if the estimated value is greater than the reference value and if the IMT value has turned out to be a reliable one.

In the example described above, if the estimated value is greater than the predetermined reference value, the IMT value turns out to be a reliable one. Depending on how to set the estimated value, however, the IMT value may also be regarded as a reliable one if the estimated value is less than the reference value. For example, the sign of the estimated value may be simply inverted. In that case, to change the reference value into a stricter one means changing the reference value into a smaller value.

In this processing step **S111** of determining whether or not to make an automatic freeze, if it has turned out in Step **S109** that it is still within a predetermined period of time since a predetermined operating event was accepted, the decision is made with the automatic freeze condition that has been modified in the previous processing step **S110** taken into account. That is to say, if the automatic freeze is disabled in Step **S111,** the automatic freeze is not made irrespective of the result of decision made by the reliability decision section **6.** Also, if the reference value to make the automatic freeze has been changed in Step **S110,** the decision is made by comparing the estimated value indicating the degree of reliability of the IMT value to the reference value that has been changed.

If the decision has been made in Step **S111** that the automatic freeze needs to be made, then the automatic freeze control section **70** determines the IMT value to be the IMT measured value in Step **S112,** thereby making a transition to the frozen state. And on entering the frozen state, the control section **7** controls the respective blocks. Specifically, the control section **7** instructs the ultrasonic wave transmission and reception processing section **2** to stop the ultrasonic wave transmission or reception processing. Also, the control section **7** controls the image synthesizing section **11** so that the tomographic image presented on the display **12** is put into a pause. In this description, "to put a tomographic image into a pause" means that the image synthesizing section **11** that has generated a synthetic signal so that a tomographic image from which the IMT measured value has been obtained is presented on the display **12** no longer updates the synthetic signal in following frames of the tomographic image.

On the other hand, if the decision has been made in Step **S111** that the automatic freeze will not be made, then the process goes back to the processing step **S101** to continue the measurement. In that case, the automatic freeze condition is reset into the original one before the condition is modified. That is to say, the automatic freeze condition is reset into the initial condition.

In Step **S113,** the image synthesizing section **11** synthesizes together the IMT measured value, which has been regarded as the final result of measurement in accordance with the decision made by the reliability decision section **6,** and the tomographic image that has been built by the tomographic image building section **31** and then outputs a synthetic image thus obtained to the display **12.** As a result, the operator can confirm the diagnostic image and the result of measurement on the screen. In this processing step, the IMT value and the tomographic image may be retrieved from the cine memory **8,** synthesized together, and their synthetic image may be displayed.

In the example illustrated in FIG. **5****,** the vascular center determining processing step **S105** and the pulsating status checkout processing step **S106** are supposed to be carried out in this order. However, these two processing steps may be performed in reverse order, too. Also, in FIG. **5****,** every time the degree of reliability of the IMT value has been determined in Step **S107,** if it is still within a predetermined period of time since a predetermined event was made, the automatic freeze condition is supposed to be modified in Steps **S109** and **S110** for convenience sake. However, event driven processing may also be carried out. Specifically, in that case, when a predetermined operating event is accepted, the automatic freeze condition is modified. Thereafter, the automatic freeze condition may be restored into the original one after a predetermined period of time has passed by performing timer processing. Or the automatic freeze condition may be gradually changed into the original one by performing timer processing in a predetermined period. That is to say, as shown in FIG. **10(a)** or **10(b)****,** the automatic freeze condition may have a reference value that changes with time. In that case, in the flowchart shown in FIG. **5****,** the processing steps **S109** and **S110** are omitted.

Also, in the embodiment described above, the vascular center determining section **32** determines, based on the tomographic image that has been generated by the tomographic image building section **31** and the vascular boundaries that have been detected by the boundary detecting section **41,** whether or not the probe **1** is put in a right position to catch the blood vessel of the subject around its center just as intended. However, if the apparatus is designed so that the received echo signal is output from the ultrasonic wave transmission and reception processing section **2** directly to the vascular center determining section **32** without passing through the tomographic image building section **31,** the amplitude of the received echo signal supplied from the ultrasonic wave transmission and reception processing section **2** may be used instead of the tomographic image to determine whether or not the probe **1** is put in a right position to catch the blood vessel around its center. In that case, the decision can be made without depending on the settings or parameters used when the tomographic image is generated.

In the example described above, a configuration for measuring an IMT value using an ultrasonic diagnostic apparatus has been described. However, the present invention can also be used for any of various other purposes, not just to measure the IMT value.

The present invention is applicable to measuring a non-IMT value by restricting the automatic freeze for only a certain period of time after a predetermined operation has been accepted when the automatic freeze is being made based on a result of the reliability decision. For example, an obstetrician measures the length of an embryo's thigh bone using an ultrasonic diagnostic apparatus. If it has been determined that the length of the thigh bone has been measured with a high degree of reliability, the automatic freeze that is going to be set off may be restricted for only a certain period of time after a predetermined operation has been accepted. In that case, the conditions may be whether the length of the thigh bone that has been obtained based on the received signal falls within a predetermined range and whether the intensity ratio of the received signal between the thigh bone and the surrounding tissue is equal to or greater than a predetermined value, for example, and the reliability decision section may decide if these conditions are satisfied.

In addition, the present invention is applicable to not just measuring something but also setting off an automatic freeze when the quality of the image obtained turns out to be high enough by a predetermined standard by restricting the automatic freeze for only a certain period of time after a predetermined operation has been accepted. In that case, the reliability decision section may determine whether the quality of the tomographic image obtained based on the received signal is high or not by calculating a numerical value representing a change of the image between frames and by seeing if that numerical value falls within a predetermined range. This is because if the probe is put on the subject in a stabilized position, there will be little change between frames and a tomographic image of quality can be obtained.

Furthermore, in the foregoing description of an embodiment to be applied to measuring an IMT value, the range to be subjected to the measuring process is supposed to be an ROI. However, a range to be subjected to ultrasonic wave transmission and reception scanning when a tomographic image or color flow image is displayed or a range to be subjected to signal processing for generating a tomographic image or a color image may also be called an "ROI". Still alternatively, the Doppler spectrum analysis range may also be called an "ROI". The present invention is applicable to any of these situations by restricting the automatic freeze for only a predetermined period of time after the ROI has been moved or its size has been changed.

Furthermore, in the foregoing description of an embodiment to be applied to measuring an IMT value, the automatic freeze is supposed to be restricted after the depth range of the tomographic image has been changed. However, the ultrasonic image, of which the depth range is changed, does not have to be a tomographic image but may also be a color flow image, an image changing with time (such as an M mode image) or any other ultrasonic image. And the present invention is applicable to any of those situations.

Some applications of the present invention other than measuring the IMT value have been described briefly. However, these are just examples and the present invention is also applicable to any other situation as long as the basic idea of the present invention is complied with. That is to say, an ultrasonic diagnostic apparatus that sets off an automatic freeze when a predetermined condition is satisfied has only to be controlled to restrict the automatic freeze for only a certain period of time after a predetermined operating event has been accepted.

Consequently, the present invention provides an ultrasonic diagnostic apparatus that comes in handier by setting off automatic freeze and yet does not have its operability affected by the automatic freeze that is set off unintentionally during various kinds of adjustments.

### INDUSTRIAL APPLICABILITY

According to the present invention with such a configuration, even while the operator is operating the measuring range or making any other adjustments, the automatic freeze is never set off at an inappropriate timing, thus improving the operability significantly. For that reason, the present invention can be used extensively in an ultrasonic diagnostic apparatus that makes various kinds of inspections and measurements (such an IMT measuring), to which the automatic freeze is applicable effectively, and a method for controlling such an ultrasonic diagnostic apparatus.

### REFERENCE SIGNS LIST

- **1**: probe
- **2**: ultrasonic wave transmission and reception processing section
- **3**: tomographic image processing section
- **4**: vascular wall thickness calculating section
- **5**: pulsation detecting section
- **6**: reliability decision section
- **7**: control section
- **8**: cine memory
- **9**: operating event accepting section
- **10**: interface
- **11**: image synthesizing section
- **12**: display
- **21**: signal processing section
- **31**: tomographic image building section
- **32**: vascular center determining section
- **41**: boundary detecting section
- **42**: IMT calculating section
- **51**: pulsation information processing section
- **52**: pulsating status checking section
- **53**: cardiac cycle detecting section
- **70**: automatic freeze control section
- **90**: blood vessel
- **91**: subject's skin surface
- **100**: controller
- **101**: ultrasonic diagnostic apparatus
- **201**: vascular lumen
- **202**: adventitia
- **203**: intima-media complex
- **204**: lumen-intima boundary
- **205**: media-adventitia boundary
- **206**: IMT measuring range
- **207**: common carotid artery
- **208**: recommended IMT measuring range

## Claims

1. An ultrasonic diagnostic apparatus to which a probe with transducers is connectible,
the apparatus comprising a controller which performs transmission processing for sending out an ultrasonic wave toward a subject by driving the probe and reception processing for generating a received signal based on the ultrasonic wave that has been reflected from the subject and received at the probe and which performs automatic freeze processing for stopping, if a predetermined condition based on the received signal is satisfied, at least one of the transmission processing and the reception processing,
wherein the controller restricts the automatic freeze processing for a predetermined period of time after having accepted a predetermined operating event.

2. An ultrasonic diagnostic apparatus to which a probe with transducers is connectible,
the apparatus comprising a controller which performs transmission processing for sending out an ultrasonic wave toward a subject by driving the probe and reception processing for generating a received signal based on the ultrasonic wave that has been reflected from the subject and received at the probe, which performs predetermined signal processing on the received signal, and which performs, if a predetermined condition based on the received signal is satisfied, automatic freeze processing for stopping at least one of the transmission processing, the reception processing and the predetermined signal processing,
wherein the controller restricts the automatic freeze processing for a predetermined period of time after having accepted a predetermined operating event.

3. The ultrasonic diagnostic apparatus of claim 1 or 2, wherein to restrict the automatic freeze processing, the controller disables the automatic freeze processing for the predetermined period of time.

4. The ultrasonic diagnostic apparatus of claim 1 or 2, wherein to restrict the automatic freeze processing, the controller performs processing for changing settings of the predetermined condition into a higher standard during the predetermined period of time.

5. The ultrasonic diagnostic apparatus of claim 1 or 2, wherein to restrict the automatic freeze processing, the controller performs processing for changing settings of the predetermined condition into a higher standard after having accepted the predetermined operating event and then restoring original settings of the predetermined condition in the predetermined period of time.

6. The ultrasonic diagnostic apparatus of one of claims 1 to 5, wherein the predetermined operating event is to terminate the frozen state.

7. The ultrasonic diagnostic apparatus of claim 2, wherein the controller performs range specifying processing for specifying at least one of the range to carry out the ultrasonic wave transmission and reception processing and the range to perform the predetermined signal processing on the received signal and wherein the predetermined operating event is to move the range or change the size of the range.

8. The ultrasonic diagnostic apparatus of claim 2, wherein the predetermined signal processing includes generating an ultrasonic image, and
wherein the predetermined operating event is to change the depth range of the ultrasonic image.

9. The ultrasonic diagnostic apparatus of one of claims 1 to 5, wherein the predetermined operating event is to change parameters of the ultrasonic wave transmission and reception processing.

10. The ultrasonic diagnostic apparatus of claim 9, wherein the parameters of the ultrasonic wave transmission and reception processing are any of a scan line density, whether or not parallel reception is enabled, the number of waves received in parallel, a transmission frequency, a transmission power and a transmission interval.

11. The ultrasonic diagnostic apparatus of claim 2, wherein the predetermined operating event is to change parameters of the predetermined signal processing.

12. The ultrasonic diagnostic apparatus of claim 11, wherein the parameters of the predetermined signal processing are any of a gain, a dynamic range, whether or not filter processing needs to be performed, and a property of the filter processing.

13. The ultrasonic diagnostic apparatus of claim 2, wherein the controller includes a storage section on which either the received signal or data that has been subjected to the predetermined signal processing is written and wherein the predetermined operating event involves clearing the data that has been written on the storage section.

14. The ultrasonic diagnostic apparatus of claim 2, wherein the controller performs the predetermined signal processing to detect the lumen-intima and media-adventitia boundaries of a blood vessel based on the received signal, thereby calculating the distance between the two boundaries as a vascular wall thickness value.

15. The ultrasonic diagnostic apparatus of claim 14, wherein the controller performs the predetermined signal processing to decide whether the vascular wall thickness value is reliable or not and to determine, based on a result of the decision, the vascular wall thickness value to be an intima-media thickness, thereby performing automatic freeze processing.

16. The ultrasonic diagnostic apparatus of claim 15, wherein the controller decides, based on a feature of the received signal on which the lumen-intima and media-adventitia boundaries of the blood vessel have been detected, whether the vascular wall thickness value calculated is reliable or not.

17. The ultrasonic diagnostic apparatus of claim 16, wherein the feature of the received signal is at least one of the intensity of the signal and the intensity distribution of the signal.

18. The ultrasonic diagnostic apparatus of claim 15, wherein the controller generates a tomographic image based on the received signal, and decides, based on a feature on the tomographic image, whether the vascular wall thickness value calculated is reliable or not.

19. The ultrasonic diagnostic apparatus of claim 18, wherein the feature on the tomographic image is at least one of its luminance, its luminance distribution and its shape.

20. A method for controlling an ultrasonic diagnostic apparatus to which a probe with transducers is connectible, the method comprising the steps of:
(i) performing transmission processing for sending out an ultrasonic wave by driving the probe and reception processing for generating a received signal based on the ultrasonic wave that has been reflected from a subject and received at the probe;
(ii) restricting automatic freeze processing for stopping, if a predetermined condition based on the received signal is satisfied, at least one of the transmission processing and the reception processing for a predetermined period of time after having accepted a predetermined operating event; and
(iii) performing the automatic freeze processing after the predetermined period of time has passed.

21. A method for controlling an ultrasonic diagnostic apparatus to which a probe with transducers is connectible, the method comprising the steps of:
(i) performing transmission processing for sending out an ultrasonic wave by driving the probe and reception processing for generating a received signal based on the ultrasonic wave that has been reflected from a subject and received at the probe;
(ii) performing predetermined signal processing on the received signal;
(iii) restricting automatic freeze processing for stopping, if a predetermined condition based on the received signal is satisfied, at least one of the transmission processing, the reception processing and the predetermined signal processing for a predetermined period of time after having accepted a predetermined operating event; and
(iv) performing the automatic freeze processing after the predetermined period of time has passed.

22. The method of claim 20 or 21, wherein the step (i) includes performing transmission processing for sending out an ultrasonic wave toward the subject, including a blood vessel, by driving the probe and reception processing for generating a received signal based on the ultrasonic wave that has been reflected from the subject including the blood vessel and received at the probe, and
wherein the step (i) is predetermined processing that includes the steps of: detecting the lumen-intima and media-adventitia boundaries of the blood vessel based on the received signal; calculating the distance between the two boundaries as a vascular wall thickness value; deciding whether the vascular wall thickness value calculated is reliable or not; and determining, based on a result of the decision, the vascular wall thickness value to be an intima-media thickness.
